# NEUE EUROPÄISCHE PATENTSCHRIFT

(11) **EP 1 815 839 B2**
(45) Veröffentlichungstag und Bekanntmachung des Hinweises auf die Entscheidung über den Einspruch: **27.05.2020**
(45) Hinweis auf die Patenterteilung: 21.06.2017
(21) Anmeldenummer: 06025996.7
(22) Anmeldetag: 15.12.2006
(51) Int. Cl.: A61K 8/02, A61K 8/04, A61K 8/25, A61K 8/36, A61K 8/44, A61K 8/64, A61K 8/65, A61K 8/67, A61K 8/92, A61K 8/96, A61K 8/97, A61K 8/98, A61Q 5/10, A61Q 5/12

(54) **Haarfärbeverfahren**
Hair colouring method
Procédé de coloration des cheveux

(30) Priorität: 23.12.2005 DE 102005062360
(43) Veröffentlichungstag der Anmeldung: 08.08.2007
(73) Patentinhaber: Henkel AG & Co. KGaA, 40589 Düsseldorf (DE)
(72) Erfinder: Kleen, Astrid, 22763 Hamburg (DE); Hoepfl, Helmut, 22559 Hamburg (DE); Terrier, Janie, 22589 Hamburg (DE); Weishaupt, Sarah, 20355 Hamburg (DE)
(74) Vertreter: LKGLOBAL Lorenz & Kopf PartG mbB Patentanwälte

(56) Entgegenhaltungen:
- EP-A1- 1 541 120
- EP-A1- 1 674 075
- EP-A2- 1 312 346
- WO-A1-2004/050048
- WO-A1-2005/058259
- US-A1- 2003 113 280
- US-A1- 2003 121 109
- US-A1- 2004 244 126
- US-B1- 6 284 003

## Beschreibung

Die vorliegende Anmeldung betrifft ein neuartiges zwei- beziehungsweise dreistufiges Verfahren zur schonenden Färbung keratinischer Fasern, insbesondere menschlicher Haare, bei dem die Fasern unmittelbar nach der oxidativen Färbung mit einem sauer eingestellten schaumförmigen Produkt behandelt werden, sowie ein Kit, umfassend die zur Durchführung dieses Verfahrens nötigen Zubereitungen.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle.

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich durch hervorragende, lang anhaltende Färbeergebnisse aus. Für natürlich wirkende Färbungen muss üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Üblicherweise werden die Fasern nach Abschluss der farbverändernden Behandlung gründlich mit Wasser oder einer tensidhaltigen wässrigen Zubereitung gespült. Zur Verbesserung des Pflegezustandes werden die Fasern anschließend häufig mit einer konditionierenden Zubereitung behandelt. US2003/0113280 offenbart ein Verfahren zur Färbung keratinischer Fasern, wobei nach der oxidativen Färbung die Fasern mit einer Vitamin B6 enthaltenden Hair-care Lotion nachbehandelt werden. Trotz der bereits guten Qualität der erzielbaren Färbungen, bestehen, nach wie vor Wünsche hinsichtlich der erzielbaren Intensität der Färbung sowie des Pflegezustandes der Fasern.

Überraschenderweise wurde nunmehr bei den dieser Anmeldung zugrunde liegenden Arbeiten gefunden, dass deutlich intensivere Färbungen erzielt werden können, wenn die Fasern unmittelbar im Anschluss an den Färbeprozess ohne Zwischenspülung mit einem sauer eingestellten Schaum behandelt werden, der mindestens einen Pflegestoff enthält. Die resultierenden Färbungen sind nicht nur von Beginn an intensiver sondern zeichnen sich ferner durch eine verbesserte Waschechtheit aus. Weiterhin wird der Pflegezustand der erfindungsgemäß behandelten Haare als verbessert empfunden.

Ein erster Gegenstand der vorliegenden Erfindung ist daher ein Verfahren zur schonenden Färbung keratinischer Fasern, insbesondere menschlicher Haare, bei dem
- in einem ersten Schritt die Fasern einer oxidativen Färbung unterzogen werden und
- anschließend in einem zweiten Schritt mit einer sauer eingestellten schaumförmigen Zubereitung, enthaltend mindestens einen Pflegestoff (P1), behandelt werden, wobei die schaumförmige Zubereitung als Aerosol formuliert ist.

Unter keratinischen Fasern sind dabei erfindungsgemäß Pelze, Wolle, Federn und insbesondere menschliche Haare zu verstehen.

Im Rahmen des erfindungsgemäßen Verfahrens wird auf die Fasern zunächst ein oxidatives Färbemittel aufgetragen. Nach Ablauf der geplanten Einwirkzeit wird dann in die Färbecreme, d.h. ohne Zwischenspülung, in einem zweiten Schritt eine sauer eingestellte schaumförmige Zubereitung (D), enthaltend mindestens einen Pflegestoff (P1) eingearbeitet. Nach Ablauf der Gesamteinwirkzeit wird das Gemisch aus Färbecreme und Pflegeschaum ausgewaschen.

Die sauer eingestellte Zubereitung (D) weist vorzugsweise einen pH-Wert von 2 bis 7 auf, Zubereitungen mit einem pH-Wert zwischen 3 und 5 sind besonders bevorzugt.

Die Zubereitung (D) wird mit Hilfe von Treibgasen oder chemischen Treibmitteln aus einem geeigneten Behälter verschäumt und als Schaum auf die mit dem Färbemittel benetzten Fasern eingearbeitet. Dabei kann der Schaum direkt auf die Fasern appliziert werden oder zunächst in die Hände gesprüht werden. Die Einarbeitung in die Fasern kann dann anschließend direkt mit den Händen oder mittels eines Kammes erfolgen. Nach einer Einwirkzeit wird die Zubereitung dann aus den Fasern ausgespült. Die Einwirkzeit des Gemischs aus Färbecreme und Zubereitung (D) beträgt dabei üblicherweise wenige Sekunden bis zu 45 Minuten. Bevorzugt sind Einwirkzeiten zwischen 30 Sekunden und 30 Minuten. Besonders bevorzugt sind Einwirkzeiten von 1 Minute bis zu 10 Minuten.

Mithilfe der aufgeschäumten Zubereitung (D) lässt sich ein Färbeergebnis erzielen, das deutlich einheitlicher ist, als bei herkömmlichen Konfektionierungsformen.

Die erfindungsgemäße Zubereitung (D) kann mit einem Treibmittel aus einem üblichen Aerosol-Druckbehälter auf dem Haar appliziert werden.

Schaumdispenser, die ohne flüchtige Treibgase betrieben werden, sind z. B. Squeeze bottles aus komprimierbarem Kunststoff. Weiterhin können Behälter aus unterschiedlichen Materialien mit einer Pumpe versehen werden, bei denen z. B. durch poröse Körper oder Siebe Luft in die Zubereitung eingebracht und so die Zubereitung als Schaum ausgetragen wird. Weiterhin können Behälter aus unterschiedlichen Materialien mit einer Düse, z. B. einer Ventilatorstrahldüse oder einer pneumatischen Düse, ausgestattet werden, wodurch ebenfalls das Austragen einer geschäumten Zubereitung möglich ist.

Geeignete Schaumdispenser, die mit flüchtigen Treibgasen betrieben werden, sind Aerosolbehälter aus Aluminium, Weißblech, Kunststoff oder Glas, die innen beschichtet sein können, mit einem geeigneten Sprühventil. Durch das enthaltene komprimierte Treibmittel, z. B. Kohlendioxid, Isobutan, Butan, Propan, Pentan, Isopentan, Fluorkohlenwasserstoffen oder Dimethylether, wird die Zubereitung (D) beim Austreten aufgeschäumt.

Unter mechanischen Schäumvorrichtungen sind solche Vorrichtungen zu verstehen, welche das Verschäumen einer Flüssigkeit mit oder ohne Verwendung eines Treibmittels ermöglichen.

Als geeignete mechanische Schäumvorrichtung kann beispielsweise ein handelsüblicher Pumpschäumer oder ein Aerosolschaumkopf verwendet werden. Diese werden beispielsweise von der Firma Airspray unter der Handelsbezeichnung "Airfoamer" angeboten. Die Schäumvorrichtungen mit der Handelsbezeichnung "Airfoamer' sind dabei besonders bevorzugt. Insbesondere bevorzugt ist der Airfoamer mit der Typenbezeichnung G3.

Zur Ausgestaltung der Erfindung als Aerosolapplikation kann jedes entsprechende Aerosolventil verwendet werden, welches die erfindungsgemäße Sprührate ermöglicht. Die Sprührate beträgt 10 g je Sekunde. Bevorzugt sind Sprühraten von 5 g je Sekunde. Ganz besonders bevorzugt sind Sprühraten von mindestens 0,5 g je Sekunde und höchstens 3,5 g je Sekunde. Hierbei kann es vorteilhaft sein, wenn die Ventilöffnung einen Durchmesser von höchstens 0,4 mm aufweist. Eine Öffnung von 0,35 mm ist dabei bevorzugt. Ganz besonders bevorzugt sind Ventilöffnungen von höchstens 0,3 mm. Derartige Ventile können im Handel beispieiswelse von den Firmen Seaquist Perfect Dispensing GmbH oder Coster Technologie Speciali s.p.a. bezogen werden.

Weiterhin ist es erfindungswesentlich, dass die erfindungsgemäße Zubereitung (D) mindestens einen Pflegestoff (P1) enthält.

Im Rahmen einer ersten bevorzugten Ausführungsform ist der Pflegestoff (P1) ausgewählt aus Vitaminen, Provitaminen, Vitaminvorstufen sowie deren Derivate.

Dabei sind erfindungsgemäß solche Vitamine, Pro-Vitamine und Vitaminvorstufen bevorzugt, die üblicherweise den Gruppen A, B, C, E, F und H zugeordnet werden.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäß verwendeten Zubereitungen enthalten die Vitamin A-Komponente bevorzugt in Mengen von 0,05-1 Gew.%, bezogen auf die gesamte Anwendungszubereitung.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß Mitteln bevorzugt in Mengen von 0,05 bis 1 Gew.-%, bezogen auf die gesamte Anwendungszubereitung, enthalten ist.
- Vitamin B₅ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate. Die genannten Verbindungen des Vitamin B₅-Typs sind in den erfindungsgemäßen Mitteln bevorzugt in Mengen von 0,05 - 10 Gew.%, bezogen auf die gesamte Anwendungszubereitung, enthalten. Mengen von 0,1 - 5 Gew.-% sind besonders bevorzugt.
- Vitamin B₆ (Pyridoxin sowie Pyridoxamin und Pyridoxal). Die genannten Verbindungen des Vitamin B₆-Typs sind in den erfindungsgemäßen Mitteln bevorzugt in Mengen von 0,01 - 5 Gew.-%, bezogen auf die gesamte Anwendungszubereitung, enthalten. Mengen von 0,05 - 1 Gew.-% sind besonders bevorzugt.

Vitamin C (Ascorbinsäure). Vitamin C wird in den erfindungsgemäß verwendeten Mitteln bevorzugt in Mengen von 0,1 bis 3 Gew.%, bezogen auf die gesamte Anwendungszubereitung eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,05-1 Gew.%, bezogen auf die gesamte Anwendungszubereitung, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3a*S*,4*S*, 6a*R*)-2-Oxohexahydrothienol[3,4-*d*]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.-%, insbesondere in Mengen von 0,001 bis 0,01 Gew.-%, jeweils bezogen auf die gesamte Anwendungszubereitung enthalten.

Besonders bevorzugte Pflegestoffe sind Vitamine, Provitamine und Vitaminvorstufen aus den Gruppen A, B, C, E und H.

Panthenol, Pantolacton, Pyridoxin und seine Derivate sowie Nicotinsäureamid und Biotin sind ganz besonders bevorzugt.

Im Rahmen einer zweiten bevorzugten Ausführungsform ist der Pflegestoff (P1) ausgewählt aus den Pflanzenextrakten.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß bevorzugten Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Grünem Tee, Eichenrinde, Brennnessel, Lilie, Rose, Orchidee, Lindenblüten, Lavendel, Rosen, Jasmin, Neroli, Ylang-Ylang Passionsblume, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng, Ingwerwurzel und Ayurveda bevorzugt.

Besonders bevorzugt sind die Extrakte aus Grünem Tee, Eichenrinde, Brennnessel, Hamamelis, Hopfen, Kamille, Klettenwurzel, Schachtelhalm, Lindenblüten, Mandel, Aloe Vera, Kokosnuss, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng, Ingwerwurzel und Ayurveda.

Ganz besonders geeignet sind die Extrakte aus Grünem Tee, Mandel, Aloe Vera, Kokosnuss, Mango, Aprikose, Limone, Weizen, Kiwi und Melone.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Mitteln Mischungen aus mehreren, insbesondere aus zwei, verschiedenen Pflanzenextrakten einzusetzen.

Im Rahmen einer dritten Ausführungsform der vorliegenden Erfindung ist der Pflegestoff (P1) ausgewählt aus pflanzlichen Ölen. Bevorzugte Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls.

Ein weiteres erfindungsgemäß bevorzugtes Pflanzenöl kann aus den Samen der Amaranthpflanze durch Extraktions- oder Pressverfahren erhalten werden. Die Lipidfraktion der Amaranthsamen besteht im Wesentlichen aus Triglyceriden. Die Linolsäure stellt dabei neben Öl- und Palmitinsäure die Hauptkomponente in der Fettsäureverteilung des Öls dar. Daneben sind aber auch Myristinsäure, Stearinsäure, Vaccensäure, α-Linolensäure, Arachinsäure, 11-Eicosensäure und Behensäure im Fettsäurespektrum vertreten. Besonders hervorzuheben ist allerdings ein besonders hoher Anteil an Squalen (6-12%) in Amaranthsamenöl, das in anderen konventionellen Pflanzenölen nur in sehr geringen Konzentrationen zu finden ist. Zudem ist das Amaranthsamenöl reich an zahlreichen Vitamin-E-Derivaten (u.a. α-, β-, γ- und δ-Tocopherol sowie α- und β-Tocotrienol) und weist einen bemerkenswerten Anteil an den Phytosterolen Δ7-Avenasterol, Δ7-Campesterol, Δ7-Sitosterol und Δ7-Stigmasterol auf. Ein erfindungsgemäß geeignetes Öl ist beispielsweise unter der Handelsbezeichnung "Amaranth Seed Oil" von der Fa. Euro Ingredients erhältlich.

Im Rahmen einer vierten bevorzugten Ausführungsform ist der Pflegestoff (P1) ausgewählt aus Proteinhydrolysaten und deren Derivaten.

Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L-Methionin-S-Methylsulfoniumchlorid.

Selbstverständlich können erfindungsgemäß auch ß-Aminosäuren und deren Derivate wie β-Alanin, Anthranilsäure oder Hippursäure eingesetzt werden. Erfindungsgemäß bevorzugte Aminosäuren sind Serin, Glycin und Lysin.

Das Molgewicht der erfindungsgemäß einsetzbaren Proteinhydrolysate liegt zwischen 75, dem Molgewicht für Glycin, und 200000, bevorzugt beträgt das Molgewicht 75 bis 50000 und ganz besonders bevorzugt 75 bis 20000 Dalton.

Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®}(Cognis), Promois^{®}(Interorgana), Collapuron^{®}(Cognis), Nutrilan^{®}(Cognis), Gelita-Sol^{®}(Deutsche Gelatine Fabriken Stoess & Co), Lexein^{®} (Inolex), Sericin (Pentapharm) und Kerasol^{®} (Croda) vertrieben.

Erfindungsgemäß bevorzugt ist die Verwendung von Proteinhydrolysaten pflanzlichen Ursprungs, z. B. Soja-, Mandel-, Erbsen-, Kartoffel- und Weizenproteinhydrolysate. Solche Produkte sind beispielsweise unter den Warenzeichen Gluadin^{®}(Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda) und Crotein^{®} (Croda) erhältlich.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon^{®} (Cognis), Lexein^{®} (Inolex), Crolastin^{®} (Croda), Crosilk^{®} (Croda) oder Crotein^{®} (Croda) vertrieben.

Selbstverständlich umfasst die erfindungsgemäße Lehre alle isomeren Formen, wie cis - trans - Isomere, Diastereomere und chirale Isomere.

Erfindungsgemäß ist es auch möglich, eine Mischung aus mehreren Proteinhydrolysaten einzusetzen.

Die Proteinhydrolysate sind in der erfindungsgemäßen Zubereitung in Konzentrationen von 0,01 Gew. % bis zu 20 Gew. %, vorzugsweise von 0,05 Gew. % bis zu 15 Gew. % und ganz besonders bevorzugt in Mengen von 0,05 Gew. % bis zu 5 Gew. % enthalten.

Im Rahmen einer fünften bevorzugten Ausführungsform der vorliegenden Erfindung ist der Pflegestoff (P1) ausgewählt aus den Betainen der Formel (A-1). Unter einem Betain im Sinne der vorliegenden Erfindung sind Verbindungen zu verstehen, welche gleichzeitig sowohl eine Gruppierung-NR₃⁺ als auch eine Gruppierung -CR₂COO⁻enthalten, analog aufgebaute Sulfobetaine sowie Verbindungen, die eine Gruppierung -NR₃⁺ und eine Gruppierung -CH₂OH aufweisen. Diese Definition basiert auf der Definition in Römpp's Lexikon Chemie - CD-ROM Version 2.0, Stuttgart/New York, Georg Thieme Verlag 1999. Insbesondere sind unter den erfindungsgemäßen Betainen solche zu verstehen, welche der Formel (A-I) entsprechen.

R¹R²R³N⁺ - (CR⁴R⁵)ₓ - (CR⁶R⁷)_{y} - (CR⁸R⁹)_{z} - Y⁻ (A-I)

R¹ R², und R³ stehen hier unabhängig voneinander für:
   - Wasserstoff,
   - einen Methylrest,
   - einen C2 - C8 gesättigten oder ungesättigten, verzweigten oder linearen oder cyclischen Kohlenwasserstoffrest
   - R⁴, R⁵, R⁶, R⁷, R⁸ und R⁹ stehen hier unabhängig voneinander für:
   - Wasserstoff,
   - -OR¹⁰,
   - -OCOR¹¹,
   - einen Methylrest, der einen 1H-Imidazolyl-4-Substituenten tragen kann,
   - einen C2 - C8 gesättigten oder ungesättigten, verzweigten oder linearen oder cyclischen Kohlenwasserstoffrest, wobei R¹⁰ steht für Wasserstoff, -CH₃ oder einen C2 - C30 gesättigten oder ungesättigten, verzweigten oder linearen oder cyclischen Kohlenwasserstoffrest und R¹¹ steht für -CH₃ oder einen C2 - C30 gesättigten oder ungesättigten, verzweigten oder linearen oder cyclischen Kohlenwasserstoffrest,
x, y und z stehen unabhängig voneinander für eine ganze Zahl von 0 bis 12 mit der Maßgabe, daß mindestens einer der Parameter x, y oder z von 0 verschieden ist und Y⁻ steht für COO⁻ , SO₃⁻ oder eine Hydroxygruppe in Kombination mit einem physiologisch verträglichen Anion.

Unter Betainen im Sinne der Erfindung sind auch solche Substanzen zu verstehen, bei denen die genannten charakteristischen Gruppen nur bei der gelösten Substanz sowie innerhalb bestimmter pH-Bereiche der Lösung vorliegen.

Erfindungsgemäß können selbstverständlich alle physiologisch verträglichen Salze der erfindungsgemäßen Betaine, insbesondere auch die Mischsalze der Betaine eingesetzt werden. Unter Mischsalzen sind feste Lösungen verschiedener Substanzen zu verstehen. Zur allgemein anerkannten Definition von Mischkristallen als feste Lösungen sei beispielsweise auf H.R. Christen, Grundlagen der allgemeinen und anorganischen Chemie, Sauerländer und Salle, 5. Auflage, 1977, auf Seite 245 verwiesen und ausdrücklich Bezug genommen. Weiterhin werden die unterschiedlichen Arten der Mischkristallbildung, wie beispielsweise Isomorphie, Homöomorphie, Heteromorphie, statistische Mischkristallbildung auch Doppelsalzbildung genannt, Mischkristalle mit oder ohne Mischungslücke etc., gemäß der Definition aus Hollemann - Wiberg, Lehrbuch der anorganischen Chemie, Walter de Gruyter, 81. - 90. Auflage 1976, Kapitel VII, Die chemische Bindung unter cc) Die Mischkristallbildung auf Seite 114 beschrieben, erfindungsgemäß unter den Mischkristallen der erfindungsgemäßen Wirkstoffe der Formel (A-I) verstanden. Auf diese Definition wird ebenfalls ausdrücklich Bezug genommen.

Unter den Mischsalzen der Betaine können einerseits die anorganischen Mischsalze wie beispielsweise Hydrochloride, Hydrobromide, Hydroiodide, Sulfate, Sulfite, Hydrogensulfate, Hydrogensulfite, Carbonate und Hydrogencarbonate, Mono-, Di-, Triphosphate oder Mischungen der Phosphate sowie Gemische dieser Mischsalze der erfindungsgemäßen Betaine verwendet werden. Andererseits können die Mischsalze der erfindungsgemäßen Betaine mit organischen Carbonsäuren, insbesondere mit den organischen mehrfunktionellen Carbonsäuren Verwendung finden. Dabei kann es bevorzugt sein, wenn die erfindungsgemäß eingesetzten mehrfunktionellen Carbonsäuren neben mindestens einer Carboxygruppe zusätzlich mindestens eine Hydroxygruppe und/oder mindestens eine Aminogruppe aufweisen. Beispiele für diese organischen Carbonsäuren sind Citronensäure, Milchsäure, Benzoesäure, Weinsäure etc, welche den Genußsäuren zuzurechnen sind. Weiterhin zählen Aminosäuren wie beispielsweise Histidin, Arginin, Lysin, Citrullin etc. zu den mehrfunktionellen organischen Säuren, welche als Mischsalze mit den erfindungsgemäßen Wirkstoffen (A) eingesetzt werden können. Erfindungsgemäß kann es bevorzugt sein, die Mischsalze in fester Form in die Formulierungen einzuarbeiten. Es ist jedoch selbstverständlich auch möglich, die Mischsalze in Form ihrer einzelnen Komponenten zu verwenden. Zur Herstellung der Mischsalze der erfindungsgemäßen Wirkstoffe der Formel (A-1) sei beispielhaft auf die US - Patentschrift 5,073,376, die europäische Anmeldeschrift EP 0 434 088 A1 oder die US Patentschrift 5,071,874 verwiesen. Das Mischungsverhältniss der erfindungsgemäßen Mischsalze kann dabei bezogen auf die jeweiligen Molmassen der einzelnen Komponenten (erfindungsgemäßes Betain der Formel (A-I) / Mischsalz bildende Substanz) zwischen 1: 50 und 50 : 1, bevorzugt zwischen 10 : 1 und 1 : 10 und ganz besonders bevorzugt zwischen 3 : 1 und 1 : 3 betragen.

Als Beispiele für die erfindungsgemäßen Wirkstoffe der Formel (A-I) und deren erfindungsgemäßen Derivate sowie Mischsalze sind zu nennen: Carnitin, Carnitintartrat, Carnitin Magnesiumcitrat, Acetylcarnitin, 3-O-Lauroyl-L-carnitin-hydrochlorid, 3-O-Octanoyl-L-carnitin-hydrochlorid, 3-O-Palmitoyl-L-carnitin-hydrochlorid, Taurin, Taurinlysylat, Taurintartrat, Taurinornithat, Lysyltaurin und Ornithyltaurin, Betalaine, 1,1-Dimethyl-Prolin, Hercynin (N□,N□,N□-Trimethyl-L-histidinium-betain), Ergothionein (Thionein, 2-Mercapto-N□, N□, N□-trimethyl-L-histidinium-betain), Cholin, Cholinchlorid, Cholinbitartrat, Cholindihydrogencitrat und die in der Literatur als Betain bezeichnete Verbindung N,N,N-trimethylglycin. Die Mischsalze können erfindungsgemäß bevorzugt sein. Ganz besonders bevorzugt kann es sein die Mischsalze der erfindungsgemäßen Wirkstoffe der Formel (A-I) und den organischen Carbonsäuren zu verwenden.

Es können erfindungsgemäß alle Arten von Isomeren, wie beispielsweise Diastereomere, Enantiomere, cis - trans-Isomere, optische Isomere, Konformationsisomere und Racemate verwendet werden.

Bevorzugt werden Carnitin, Taurin, Histidin, Cholin, Betain sowie deren Derivate. Dabei können die erfindungsgemäßen Mittel sowohl eine Verbindung gemäß Formel (A-I) als auch mehrere, insbesondere zwei, Verbindungen der Formel (A-I) enthalten. Ganz besonders bevorzugt kann es sein, die Mischsalze der erfindungsgemäßen Wirkstoffe der Formel (A-I) einzusetzen.

Die erfindungsgemäßen Mittel enthalten die Betaine in Mengen von 0,001 bis 20 Gew.-%, bezogen auf das gesamte Mittel. Ein Gehalt von 0,05 bis 10 Gew.-% ist bevorzugt.

Im Rahmen einer sechsten bevorzugten Ausführungsform der vorliegenden Erfindung ist der Pflegestoff (P1) ausgewählt aus den Extrakten, die aus Perlen gewonnen werden können.

Perlen von Muscheln bestehen im Wesentlichen aus anorganischen und organischen Calciumsalzen, Spurenelementen und Proteinen. Perlen lassen sich auf einfache Weise aus kultivierten Muscheln gewinnen. Die Kultivierung der Muscheln kann sowohl in Süßwasser als auch in Meereswasser erfolgen. Dies kann sich auf die Inhaltsstoffe der Perlen auswirken. Erfindungsgemäß bevorzugt ist ein Perlenextrakt, welcher von in Meeres- bzw. Salzwasser kultivierten Muscheln stammt. Die Perlen bestehen zu einem großen Teil aus Aragonit (Calciumcarbonat), Conchiolin und einem Albuminoid. Letztere Bestandteile sind Proteine. Weiterhin sind in Perlen noch Magnesium- und Natriumsalze, anorganische Siliciumverbindungen sowie Phosphate enthalten.

Zur Herstellung des Perlenextraktes werden die Perlen pulverisiert. Danach werden die pulverisierten Perlen mit den üblichen Methoden extrahiert. Als Extraktionsmittel zur Herstellung der Perlenextrakte können Wasser, Alkohole sowie deren Mischungen verwendet werden. Unter. Wasser sind dabei sowohl demineralisiertes Wasser, als auch Meereswasser zu verstehen. Unter den Alkoholen sind dabei niedere Alkohole wie Ethanol und Isopropanol, insbesondere aber mehrwertige Alkohole wie Glycerin, Diglycerin, Triglycerin, Polyglycerin, Ethylenglykol, Propylenglykol und Butylenglykol, sowohl als alleiniges Extraktionsmittel als auch in Mischung mit demineralisiertem Wasser oder Meereswasser, bevorzugt. Perlenextrakte auf Basis von Wasser/Glyceringemischen haben sich als besonders geeignet erwiesen. Je nach Extraktionsbedingungen können die Perlenproteine (Conchiloin und Albuminoid) weitestgehend in nativem Zustand oder bereits teilweise oder weitestgehend als Proteinhydrolysate vorliegen. Bevorzugt ist ein Perlenextrakt, in welchem Conchiolin und Albuminoid bereits teilweise hydrolysiert vorliegen. Die wesentlichen Aminosäuren dieser Proteine sind Glutaminsäure, Serin, Alanin, Glycin Asparaginsäure und Phenylalanin. In einer weiteren besonders bevorzugten Ausgestaltung kann es vorteilhaft sein, wenn der Perlenextrakt zusätzlich mit mindestens einer oder mehreren dieser Aminosäuren diesen Aminosäuren angereichert wird. In der bevorzugtesten Ausführungsform ist der Perlenextrakt angereichert mit Glutaminsäure, Serin und Leucin. Weiterhin findet sich je nach Extraktionsbedingungen, insbesondere in Abhängigkeit von der Wahl des Extraktionsmittels ein mehr oder weniger großer Anteil an Mineralien und Spurenelementen im Extrakt wieder. Ein bevorzugter Extrakt enthält organische und/oder anorganische Calciumsalze sowie Magnesium- und Natriumsalze, anorganische Siliciumverbindungen und/oder Phosphate. Ein ganz besonders bevorzugter Perlenextrakt enthält mindestens 75 %, bevorzugt 85 %, bevorzugter 90 % und ganz besonders bevorzugt 95 % aller Inhaltsstoffe der natürlich vorkommenden Perlen.
Beispiele für erfindungsgemäße Perlenextrakte sind die Handelsprodukte Pearl Protein Extract BG^{®} oder Crodarom^{®} Pearl.

In den kosmetischen Zusammensetzungen ist einer der zuvor beschriebenen Perlenextrakte in einer Menge von mindestens 0,01 bis zu 20 Gew.-% enthalten. Bevorzugt werden Mengen des Extraktes von 0,01 bis zu 10 Gew.%, ganz besonders bevorzugt Mengen von 0,01 bis 5 Gew.-% bezogen auf die gesamte kosmetische Zusammensetzung verwendet.

Im Rahmen einer siebten Ausführungsform ist der Pflegestoff (P1) ausgewählt aus den Meersalzmineralien. Den Hauptanteil der Meersalzmineralien bilden die Alkalimetallchloride, wobei Natriumchlorid die dominierende Rolle spielt. Weitere Bestandteile sind Magnesiumchlorid, Magnesiumsulfat, Calciumsulfat, Kaliumchlorid und Calciumcarbonat, aber auch andere Salze sind in Spuren im Meerwasser gelöst. Erfindungsgemäß besonders bevorzugte Meersalzmineralien sind Natriumchlorid, Magnesiumsulfat, Magnesiumchlorid, Calciumchlorid und Natriumhydrogencarbonat.

Im Rahmen einer achten Ausführungsform ist der Pflegestoff (P1) ausgewählt aus den Edelsteinen. Erfindungsgemäß kann es besonders bevorzugt sein, dass die Zubereitung (D) Spuren eines Edelsteines enthält. Besonders bevorzugte Edelsteine sind Rubin, Rosenquarz, Bergkristall und Saphir.

Im Rahmen der vorliegenden Erfindung hat es sich als besonders vorteilhaft herausgestellt, wenn der Pflegestoff (P1) ausgewählt ist aus Carnitin und seinen Salzen, insbesondere Carnitintatrat, Aminosäuren, insbesondere Serin, Lysin und Glycin, Extrakten, die aus Perlen gewonnen werden können, Bestandteilen, die aus Edelsteinen gewonnen werden können, Meersalzmineralien, Pflanzenölen, Pflanzenextrakten und/oder Vitaminen.

Carnitintatrat und Serin sind ganz besonders bevorzugte Pflegestoffe (P1).

Im Rahmen eines zweiten Gegenstandes des erfindungsgemäßen Verfahrens werden die Fasern im Rahmen des ersten Verfahrensschrittes mit einer Zubereitung behandelt, die unmittelbar vor der Anwendung aus einer ersten Zubereitung (A), enthaltend mindestens ein Oxidationsfarbstoffvorprodukt, und einer zweiten Zubereitung (B), enthaltend mindestens ein Oxidationsmittel, hergestellt wird.

Die erfindungsgemäßen Zubereitungen enthalten als Oxidationsfarbstoffvorprodukt vorzugsweise mindestens eine Entwicklerkomponente. Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolderivate sowie 2,4,5,6-Tetraaminopyrimidin und dessen Derivate eingesetzt.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1) 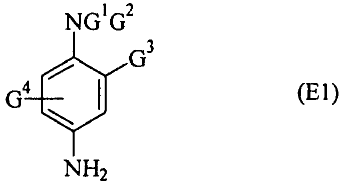 wobei
- G¹ steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen 4'-Aminophenylrest oder einen C₁- bis C₄-Alkylrest, der mit einer stickstoffhaltigen Gruppe, einem Phenyl- oder einem 4'-Aminophenylrest substituiert ist;
- G² steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C4)-Alkoxy-(C₁- bis C₄)-alkylrest oder einen C₁- bis C₄-Alkylrest, der mit einer stickstoffhaltigen Gruppe substituiert ist;
- G³ steht für ein Wasserstoffatom, ein Halogenatom, wie ein Chlor-, Brom-, lod- oder Fluoratom, einen C₁- bis C₄-Alkylrest, einen C₁- bis C₄-Monohydroxyalkylrest, einen C₂- bis C₄-Polyhydroxyalkylrest, einen C₁- bis C₄-Hydroxyalkoxyrest, einen C₁- bis C₄-Acetylaminoalkoxyrest, einen C₁- bis C₄- Mesylaminoalkoxyrest oder einen C₁- bis C₄-Carbamoylaminoalkoxyrest;
- G⁴ steht für ein Wasserstoffatom, ein Halogenatom oder einen C₁- bis C₄-Alkylrest oder
- wenn G³ und G⁴ in ortho-Stellung zueinander stehen, können sie gemeinsam eine verbrückende α,ω-Alkylendioxogruppe, wie beispielsweise eine Ethylendioxygruppe bilden.

Beispiele für die als Substituenten in den erfindungsgemäßen Verbindungen genannten C₁- bis C₄-Alkylreste sind die Gruppen Methyl, Ethyl, Propyl, Isopropyl und Butyl. Ethyl und Methyl sind bevorzugte Alkylreste. Erfindungsgemäß bevorzugte C₁- bis C₄-Alkoxyreste sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (E1) sind insbesondere die Aminogruppen, C₁- bis C₄-Monoalkylaminogruppen, C₁- bis C₄-Dialkylaminogruppen, C₁- bis C₄-Trialkylammoniumgruppen, C₁- bis C₄-Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)-amino-2-methylanilin, 4-N,N-Bis-(β-hydroxyethyl)-amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin, N-(4-Amino-3-methylphenyl)-N-[3-(1H-imidazol-1-yl)propyl]amin und 5,8-Diaminobenzo-1,4-dioxan sowie ihren physiologisch verträglichen Salzen.

Erfindungsgemäß ganz besonders bevorzugte p-Phenylendiaminderivate der Formel (E1) sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze: 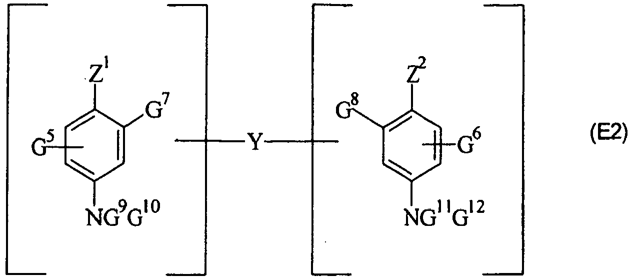 wobei:
- Z¹ und Z² stehen unabhängig voneinander für einen Hydroxyl- oder NH₂-Rest, der gegebenenfalls durch einen C₁- bis C₄-Alkylrest, durch einen C₁- bis C₄-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁- bis C₈-Alkoxyreste substituiert sein kann, oder eine direkte Bindung,
- G⁵ und G⁶ stehen unabhängig voneinander für ein Wasserstoff- oder Halogenatom, einen C₁- bis C₄-Alkylrest, einen C₁- bis C₄-Monohydroxyalkylrest, einen C₂- bis C₄- Polyhydroxyalkylrest, einen C₁- bis C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁- bis C₄-Alkylrest,
mit der Maßgabe, dass die Verbindungen der Formel (E2) nur eine Verbrückung Y pro Molekül enthalten.

Die in Formel (E2) verwendeten Substituenten sind erfindungsgemäß analog zu den obigen Ausführungen definiert.

Bevorzugte zweikernige Entwicklerkomponenten der Formel (E2) sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (E2) sind N,N'-Bis-(β-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan und 1,10-Bis-(2,5-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 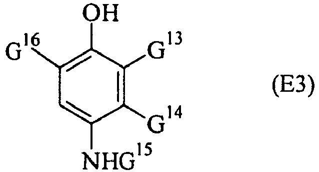 wobei:
- G¹³ steht für ein Wasserstoffatom, ein Halogenatom, einen C₁- bis C₄-Alkylrest, einen C₁-bis C₄-Monohydroxyalkylrest, einen C₂- bis C₄-Polyhydroxyalkylrest, einen (C₁- bis C₄)-Alkoxy-(C₁-bis C₄)-alkylrest, einen C₁- bis C₄-Aminoalkylrest, einen Hydroxy-(C₁- bis C₄)-alkylaminorest, einen C₁- bis C₄-Hydroxyalkoxyrest, einen C₁- bis C₄-Hydroxyalkyl-(C₁-bis C₄)-aminoalkylrest oder einen (Di-C₁-bis C₄-Alkylamino)-(C₁-bis C₄)-alkylrest, und
- G¹⁴ steht für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁-bis C₄)-alkylrest, einen C₁- bis C₄-Aminoalkylrest oder einen C₁- bis C₄-Cyanoalkylrest,
- G¹⁵ steht für Wasserstoff, einen C₁- bis C₄-Alkylrest, einen C₁- bis C₄-Monohydroxyalkylrest, einen C₂- bis C₄-Polyhydroxyalkylrest, einen Phenylrest oder einen Benzylrest, und
- G¹⁶ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-, Pyrazol-PyrimidinDerivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4-Methoxyphenyl)-amino-3-amino-pyridin, 2,3-Diamino-6-methoxy-pyridin, 2-(β-Methoxyethyl)-amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740 931 und DE 195 43 988 beschrieben werden, wie 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)-amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl)-amino-1-methylpyrazol.

Bevorzugte Pyrazolopyrimidin-Derivate sind insbesondere die Derivate des Pyrazolo[1,5-a]pyrimidin der folgenden Formel (E4) und dessen tautomeren Formen, sofern ein tautomeres Gleichgewicht besteht: 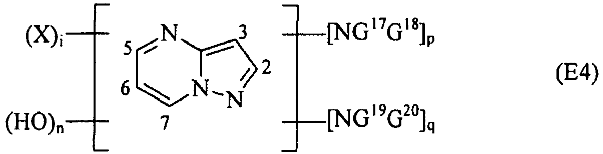 wobei:
- G¹⁷, G¹⁸, G¹⁹ und G²⁰ unabhängig voneinander stehen für ein Wasserstoffatom, einen C₁-bis C₄-Alkylrest, einen Aryl-Rest, einen C₁- bis C₄-Hydroxyalkylrest, einen C₂- bis C₄-Polyhydroxyalkylrest einen (C₁- bis C₄)-Alkoxy-(C₁- bis C₄)-alkylrest, einen C₁- bis C₄-Aminoalkylrest, der gegebenenfalls durch ein Acetyl-Ureid- oder einen Sulfonyl-Rest geschützt sein kann, einen (C₁- bis C₄)-Alkylamino-(C₁- bis C₄)-alkylrest, einen Di-[(C₁- bis C₄)-alkyl]-(C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄)-[Hydroxyalkyl]-(C₁- bis C₄)-aminoalkylrest,
- die X-Reste stehen unabhängig voneinander für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest, einen C₁- bis C₄-Aminoalkylrest, einen (C₁- bis C₄)-Alkylamino-(C₁- bis C₄)-alkylrest, einen Di-[(C₁- bis C₄)alkyl]- (C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄-hydroxyalkyl)-aminoalkylrest, einen Aminorest, einen C₁- bis C₄-Alkyl- oder Di-(C₁- bis C₄-hydroxyalkyl)-aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
mit der Maßgabe, dass
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (E4) verwendeten Substituenten sind erfindungsgemäß analog zu den obigen Ausführungen definiert.

Wenn das Pyrazolo[1,5-a]-pyrimidin der obenstehenden Formel (E4) eine Hydroxygruppe an einer der Positionen 2, 5 oder 7 des Ringsystems enthält, besteht ein tautomeres Gleichgewicht, das zum Beispiel im folgenden Schema dargestellt wird:

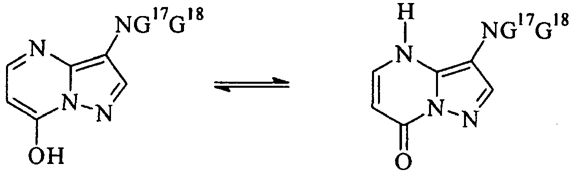

Unter den Pyrazolo[1,5-a]-pyrimidinen der obenstehenden Formel (E4) kann man insbesondere nennen:
- Pyrazolo[1,5-a]pyrimidin-3,7-diamin;
- 2,5-Dimethyl-pyrazolo[1,5-a]pyrimidin-3,7-diamin;
- Pyrazolo[1,5-a]pyrimidin-3,5-diamin;
- 2,7-Dimethyl-pyrazolo[1,5-a]pyrimidin-3,5-diamin;
- 3-Aminopyrazolo[1,5-a]pyrimidin-7-ol;
- 3-Aminopyrazolo[1,5-a]pyrimidin-5-ol;
- 2-(3-Aminopyrazolo[1,5-a]pyrimidin-7-ylamino)-ethanol;
- 2-(7-Aminopyrazolo[1,5-a]pyrimidin-3-ylamino)-ethanol;
- 2-[(3-Aminopyrazolo[1,5-a]pyrimidin-7-yl)-(2-hydroxy-ethyl)-amino]-ethanol;
- 2-[(7-Aminopyrazolo[1,5-a]pyrimidin-3-yl)-(2-hydroxy-ethyl)-amino]-ethanol;
- 5,6-Dimethylpyrazolo[1,5-a]pyrimidin-3,7-diamin;
- 2,6-Dimethylpyrazolo[1,5-a]pyrimidin-3,7-diamin;
- 3-Amino-7-dimethylamino-2,5-dimethylpyrazolo[1,5-a]pyrimidin;
sowie ihre physiologisch verträglichen Salze und ihre tautomeren Formen, wenn ein tautomers Gleichgewicht vorhanden ist.

Die Pyrazolo[1,5-a]pyrimidine der obenstehenden Formel (E4) können wie in der Literatur beschrieben durch Zyklisierung ausgehend von einem Aminopyrazol oder von Hydrazin hergestellt werden.

Neben oder anstelle der mindestens einen Entwicklerkomponente kann die erfindungsgemäße Zubereitung (A) als Oxidationsfarbstoffvorprodukt auch mindestens eine Kupplerkomponente enthalten.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Erfindungsgemäß bevorzugte Kupplerkomponenten sind
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxy-ethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]2-methoxy-5-methylphenyl}amino)ethanol, 2-({3-[(2-Hydroxyethyl)amino]-4,5-dimethylphenyl}-amino)ethanol, 2-[3-Morpholin-4-ylphenyl)amino]ethanol, 3-Amino-4-(2-methoxyethoxy)-5-methylphenylamin und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Amino-benzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol sowie deren physiologisch verträglichen Salze.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Die erfindungsgemäßen Zubereitungen enthalten sowohl die Entwicklerkomponenten als auch die Kupplerkomponenten bevorzugt in einer derartigen Menge, dass bezogen auf das gesamte Oxidationsfärbemittel (Mischung aus den Zubereitungen (A) und (B) sowie ggf. (C) eine Menge von 0,005 bis 20 Gew.-%, vorzugsweise 0,1 bis 5 Gew.-% resultiert. Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, enthalten sein können.

Neben dem erfindungswesentlichen mindestens einem Oxidationsfarbstoffvorprodukt kann die Zubereitung (A) der vorliegenden Erfindung weiterhin mindestens eine Vorstufe eines naturanalogen Farbstoffs enthalten. Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe.

Besonders gut als Vorstufen naturanaloger Haarfarbstoffe geeignet sind Derivate des 5,6-Dihydroxyindolins der Formel (NAV I), 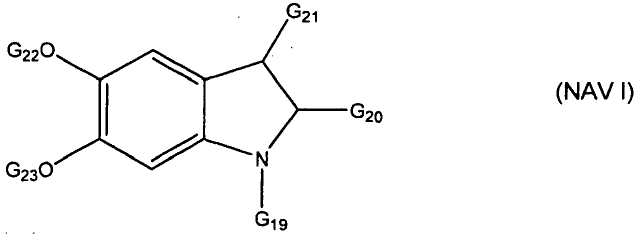 in der unabhängig voneinander
- G₁₉ steht für Wasserstoff, eine C₁-C₄-Alkylgruppe oder eine C₁-C₄-Hydroxy-alkylgruppe,
- G₂₀ steht für Wasserstoff oder eine -COOH-Gruppe, wobei die -COOH-Gruppe auch als Salz mit einem physiologisch verträglichen Kation vorliegen kann,
- G₂₁ steht für Wasserstoff oder eine C₁-C₄-Alkylgruppe,
- G₂₂ steht für Wasserstoff, eine C₁-C₄-Alkylgruppe oder eine Gruppe -CO-G₂₄, in der G₂₄ steht für eine C₁-C₄-Alkylgruppe, und
- G₂₃ steht für eine der unter G₂₂ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbon-säure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (NAV II), 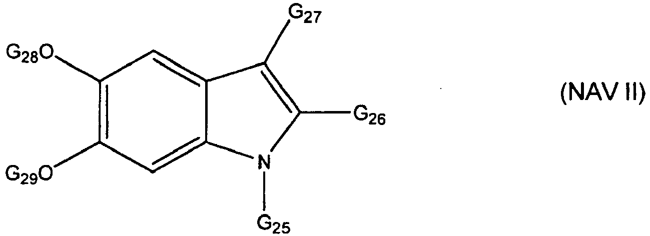 in der unabhängig voneinander
- G₂₅ steht für Wasserstoff, eine C₁-C₄-Alkylgruppe oder eine C₁-C₄-Hydroxyalkylgruppe,
- G₂₆ steht für Wasserstoff oder eine -COOH-Gruppe, wobei die -COOH-Gruppe auch als Salz mit einem physiologisch verträglichen Kation vorliegen kann,
- G₂₇ steht für Wasserstoff oder eine C₁-C₄-Alkylgruppe,
- G₂₈ steht für Wasserstoff, eine C₁-C₄-Alkylgruppe oder eine Gruppe -CO-G₃₀, in der G₃₀ steht für eine C₁-C₄-Alkylgruppe, und
- G₂₉ steht für eine der unter G₂₈ genannten Gruppen,
- sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den erfindungsgemäßen Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

In einer weiteren Ausführungsform kann es erfindungsgemäß bevorzugt sein, das Indolin- oder Indolderivat in Färbemitteln in Kombination mit mindestens einer Aminosäure oder einem Oligopeptid einzusetzen. Die Aminosäure ist vorteilhafterweise eine α-Aminosäure; ganz besonders bevorzugte α-Aminosäuren sind Arginin, Ornithin, Lysin, Serin und Histidin, insbesondere Arginin.

Weiterhin kann die Zubereitung (A) zur weiteren Nuancierung der resultierenden Färbung mindestens einen direktziehenden Farbstoff enthalten. Die direktziehenden Farbstoffe sind vorzugsweise ausgewählt aus den Nitrophenylendiaminen, den Nitroaminophenolen, den Azofarbstoffen, den Anthrachinonen oder den Indophenolen. Besonders bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner kann es erfindungsgemäß bevorzugt sein, dass die Mittel mindestens einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die mindestens einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

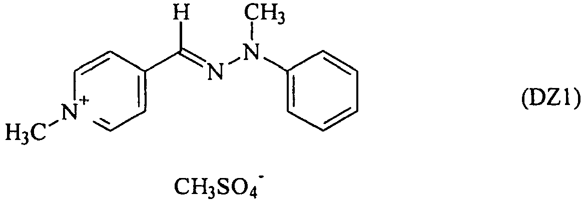

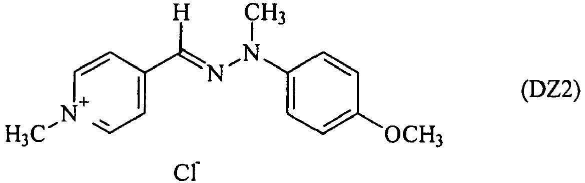

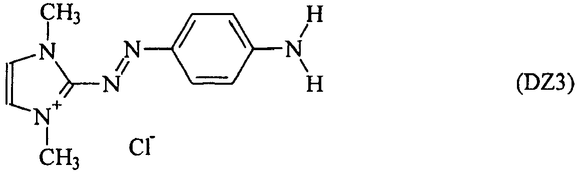

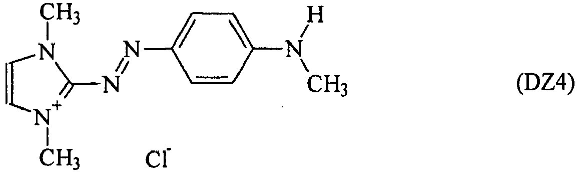

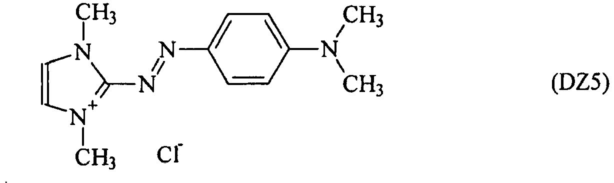

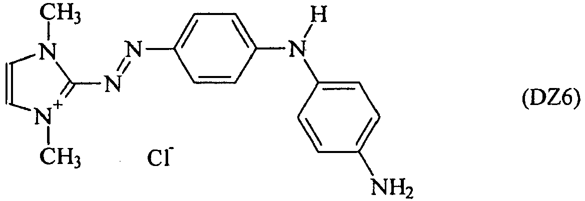

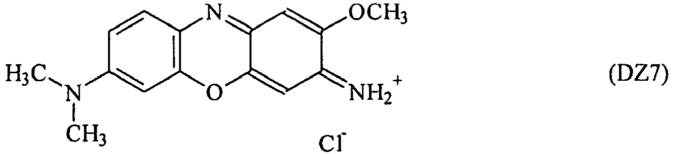

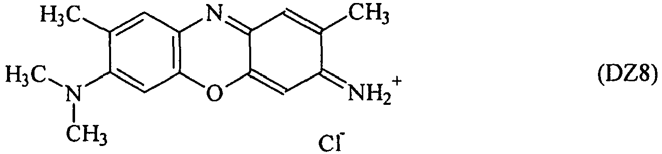

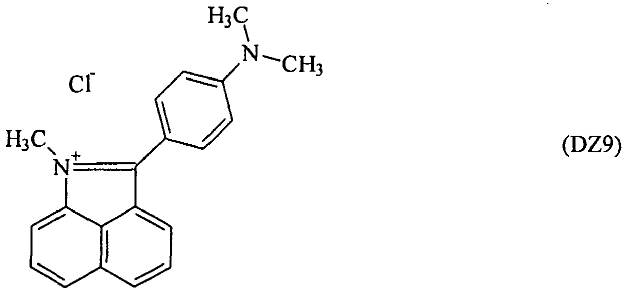

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe.

Weiterhin können die erfindungsgemäßen Zubereitungen auch in der Natur vorkommende direktziehende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Die erfindungsgemäßen Mittel enthalten die direktziehenden Farbstoffe bevorzugt in einer Menge von 0,01 bis 20 Gew.-%, bezogen auf die gesamte Anwendungszubereitung (Mischung aus den Zubereitungen (A) und (B) sowie ggf. (C)).

Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Die erfindungsgemäße Zubereitung (A) enthält das mindestens eine Oxidationsfarbstoffvorprodukt bevorzugt in einem geeigneten wässrigen, alkoholischen oder wässrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch denkbar, die Oxidationsfarbstoffvorprodukte in eine pulverförmige oder auch Tabletten-förmige Formulierung zu integrieren.

Unter wässrig-alkoholischen Lösungen sind im Sinne der vorliegenden Erfindung wäßrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Hinsichtlich der weiteren fakultativen Komponenten der Zubereitung (A) sei an dieser Stelle auf die folgenden allgemeinen Ausführungen verwiesen.

Im Rahmen des erfindungsgemäßen Verfahrens wird die Anwendungszubereitung zur oxidativen Färbung unmittelbar vor der Anwendung aus einer ersten Zubereitung (A), enthaltend mindestens ein Oxidationsfarbstoffvorprodukt, und einer zweiten Zubereitung (B), enthaltend mindestens ein Oxidationsmittel hergestellt.

Als Oxidationsmittel kommen erfindungsgemäß vorzugsweise Persulfate, Chlorite und insbesondere Wasserstoffperoxid oder dessen Anlagerungsprodukte an Harnstoff, Melamin sowie Natriumborat in Frage. Erfindungsgemäß kann die Zubereitung (B) als Oxidationsmittel aber auch einen Katalysator enthalten, der die Oxidation der Farbstoffvorprodukte, z.B. durch Luftsauerstoff, aktiviert. Solche Katalysatoren sind z.B. Metallionen, lodide, Chinone oder bestimmte Enzyme.

Geeignete Metallionen sind beispielsweise Zn²⁺, Cu²⁺, Fe²⁺ , Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Das eigentliche Haarfärbemittel wird erfindungsgemäß unmittelbar vor der Anwendung durch Mischung der Zubereitung (B) mit der Zubereitung (A), enthaltend die Farbstoffvorprodukte sowie die erfindungsgemäßen Wirkstoffe, hergestellt. Das dabei entstehende gebrauchsfertige Haarfärbepräparat sollte bevorzugt einen pH-Wert im Bereich von 6 bis 12 aufweisen. Besonders bevorzugt ist die Anwendung der Haarfärbemittel in einem schwach alkalischen Milieu. Die Anwendungstemperaturen können in einem Bereich zwischen 15 und 40 °C liegen.

Die Oxidationsmittelzubereitung enthält neben dem eigentlichen Oxidationsmittel weitere Hilfs- und Zusatzstoffe. So hat es sich erfindungsgemäß als bevorzugt erwiesen, wenn die Oxidationsmittelzubereitung mindestens ein Verdickungsmittel enthält. Bezüglich dieser Verdickungsmittel bestehen keine prinzipiellen Einschränkungen. Es können sowohl organische als auch rein anorganische Verdickungsmittel zum Einsatz kommen.

Gemäß einer ersten bevorzugten Ausführungsform handelt es sich bei dem Verdickungsmittel um ein anionisches, synthetisches Polymer. Bevorzugte anionische Gruppen sind die Carboxylat- und die Sulfonatgruppe.

Beispiele für anionische Monomere, aus denen die polymeren anionischen Verdickungsmittel bestehen können, sind Acrylsäure, Methacrylsäure, Crotonsäure, Itaconsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind Maleinsäureanhydrid sowie insbesondere 2-Acrylamido-2-methylpropansulfonsäure und Acrylsäure.

Bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichnen Carbopol^{®} im Handel erhältlich. Ebenfalls bevorzugt ist das Homopolymer der 2-Acrylamido-2-methylpropansulfonsäure, das beispielsweise unter der Bezeichnung Rheothik^{®} 11-80 im Handel erhältlich ist.

Innerhalb dieser ersten Ausführungsform kann es weiterhin bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Itaconsäuremono- und -diester, Vinylpyrrolidon, Vinylether und Vinylester.

Bevorzugte anionische Copolymere sind beispielsweise Copolymere aus Acrylsäure, Methacrylsäure oder deren C₁- bis C₆-Alkylestern, wie sie unter der INCI-Deklaration Acrylates Copolymere vertrieben werden. Ein bevorzugtes Handelsprodukt ist beispielsweise Aculyn^{®} 33 der Firma Rohm & Haas. Weiterhin bevorzugt sind aber auch Copolymere aus Acrylsäure, Methacrylsäure oder deren C₁- bis C₆-Alkylestern und den Estern einer ethylenisch ungesättigten Säure und einem alkoxylierten Fettalkohol. Geeignete ethylenisch ungesättigte Säuren sind insbesondere Acrylsäure, Methacrylsäure und Itaconsäure; geeignete alkoxylierte Fettalkohole sind insbesondere Steareth-20 oder Ceteth-20. Derartige Copolymere werden von der Firma Rohm & Haas unter der Handelsbezeichnung Aculyn^{®} 22 sowie von der Firma National Starch unter den Handelsbezeichnungen Structure^{®} 2001 und Structure^{®} 3001 vertrieben.

Bevorzugte anionische Copolymere sind weiterhin Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxythan, Allylsucrose, Allylpentaerythrit und Methylen-bisacrylamid zum Einsatz kommen. Ein solches Polymer ist in den Handelsprodukten Sepigel^{®}305 und Simulgel^{®} 600 der Firma SEPPIC enthalten. Die Verwendung dieser Compounds, die neben der Polymerkomponente eine Kohlenwasserstoffmischung (C₁₃-C₁₄-Isoparaffin beziehungsweise Isohexadecan) und einen nichtionogenen Emulgator (Laureth-7 beziehungsweise Polysorbate-80) enthalten, hat sich im Rahmen der erfindungsgemäßen Lehre als besonders vorteilhaft erwiesen.

Auch Polymere aus Maleinsäureanhydrid und Methylvinylether, insbesondere solche mit Vernetzungen, sind bevorzugte Verdickungsmittel. Ein mit 1,9-Decadien vernetztes Maleinsäure-Methylvinylether-Copolymer ist unter der Bezeichnungg Stabileze^{®} QM im Handel erhältlich.

Gemäß einer zweiten Ausführungsform handelt es sich bei dem Verdickungsmittel um einen kationisches synthetisches Polymer. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (I), 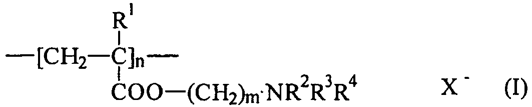 in der R¹ = -H oder -CH₃ ist, R², R³ und R⁴ unabhängig voneinander ausgewählt sind aus C₁₋₄-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel (I) aufgeführten Monomereinheiten sowie nichtionogenen Monomereinheiten, sind besonders bevorzugte kationische polymere Gelbildner. Im Rahmen dieser Polymeren sind diejenigen erfindungsgemäß bevorzugt, für die mindestens eine der folgenden Bedingungen gilt:
- R¹ steht für eine Methylgruppe
- R², R³ und R⁴ stehen für Methylgruppen
- m hat den Wert 2,

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Monnitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponente: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6) im Handel erhältlich.

Copolymere mit Monomereinheiten gemäß Formel (I) enthalten als nichtionogene Monomereinheiten bevorzugt Acrylamid, Methacrylamid, Acrylsäure-C₁₋₄-Alkylester und Methacrylsäure-C₁₋₄-Alkylester. Unter diesen nichtionogenen Monomeren ist das Acrylamid besonders bevorzugt. Auch diese Copolymere können, wie im Falle der Homopolymeren oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomeren in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

In einer dritten bevorzugten Ausführungsform werden natürlich vorkommende Verdickungsmittel eingesetzt. Bevorzugte Verdickungsmittel dieser Ausführungsform sind beispielsweise nichtionischen Guargums. Erfindungsgemäß können sowohl modifizierte als auch unmodifizierte Guargums zum Einsatz kommen. Nichtmodifizierte Guargums werden beispielsweise unter der Handelsbezeichnung Jaguar^{®} C von der Firma Rhone Poulenc vertrieben. Erfindungsgemäß bevorzugte modifizierte Guargums enthalten C₁- bis C₆-Hydroxyalkylgruppen. Bevorzugt sind die Gruppen Hydroxymethyl, Hydroxyethyl, Hydroxypropyl und Hydroxybutyl. Derart modifizierte Guargums sind im Stand der Technik bekannt und können beispielsweise durch Reaktion der Guargums mit Alkylenoxiden hergestellt werden. Der Grad der Hydroxyalkylierung, der der Anzahl der verbrauchten Alkylenoxidmoleküle im Verhältnis zur Zahl der freien Hydroxygruppen der Guargums entspricht, liegt bevorzugt zwischen 0,4 und 1,2. Derart modifizierte Guar Gums sind unter den Handelsbezeichnungen Jaguar^{®} HP8, Jaguar^{®} HP60, Jaguar^{®} HP120, Jaguar^{®} DC 293 und Jaguar^{®} HP105 der Firma Rhone Poulenc im Handel erhältlich.

Weiterhin geeignete natürliche Verdickungsmittel sind ebenfalls bereits aus dem Stand der Technik bekannt. Es wird daher explizit auf das Werk von Robert L. Davidson mit dem Titel "Handbook of Water soluble gums and resins", erschienen bei Mc Graw Hill Book Company (1980) verwiesen.

Gemäß dieser Ausführungsform bevorzugt sind weiterhin Biosaccharidgums mikrobiellen Ursprungs, wie die Skleroglucangums oder Xanthangums, Gums aus pflanzlichen Exsudaten, wie beispielsweise Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, Cellulosederivate, wie beispielsweise Methylcellulose, Carboxyalkylcellulosen und Hydroxyalkylcellulosen.

Bevorzugte Hydroxyalkylcellulosen sind insbesondere die Hydroxyethylcellulosen, die unter den Bezeichnungen Cellosize^{®} der Firma Amerchol und Natrosol^{®} der Firma Hercules vertrieben werden. Geeignete Carboxyalkylcellulosen sind insbesondere die Carboxymethylcellulosen, wie sie unter den Bezeichnungen Blanose^{®} von der Firma Aqualon, Aquasorb^{®} und Ambergum^{®} von der Firma Hercules und Cellgon^{®} von der Firma Montello vertrieben werden.

Bevorzugt sind weiterhin Stärke und deren Derivate. Stärke ist ein Speicherstoff von Pflanzen, der vor allem in Knollen und Wurzeln, in Getreide-Samen und in Früchten vorkommt und aus einer Vielzahl von Pflanzen in hoher Ausbeute gewonnen werden kann. Das Polysaccharid, das in kaltem Wasser unlöslich ist und in siedendem Wasser eine kolloidale Lösung bildet, kann beispielsweise aus Kartoffeln, Maniok, Bataten, Maranta, Mais, Getreide, Reis, Hülsenfrüchte wie beispielsweise Erbsen und Bohnen, Bananen oder dem Mark bestimmter Palmensorten (beispielsweise der Sagopalme) gewonnen werden. Erfindungsgemäß einsetzbar sind natürliche, aus Pflanzen gewonnene Stärken und/oder chemisch oder physikalisch modifizierte Stärken. Eine Modifizierung läßt sich beispielsweise durch Einführung unterschiedlicher funktioneller Gruppen an einer oder mehreren der Hydroxylgruppen der Stärke erreichen. Üblicherweise handelt es sich um Ester, Ether oder Amide der Stärke mit gegebenenfalls substituierten C₁- bis C₄₀-Resten. Besonders vorteilhaft ist eine mit einer 2-Hydroxypropylgruppe veretherte Maisstärke, wie sie beispielsweise von der Firma National Starch unter der Handelsbezeichnung Amaze^{®} vertrieben wird.

Aber auch nichtionische, vollsynthetische Polymere, wie beispielsweise Polyvinylalkohol oder Polyvinylpyrrolidon, sind als erfindungsgemäße Verdickungsmittel einsetzbar. Bevorzugte nichtionische, vollsynthetische Polymere werden beispielsweise von der Firma BASF unter dem Handelsnamen Luviskol^{®} vertrieben. Derartige nichtionische Polymere ermöglichen, neben ihren hervorragenden verdickenden Eigenschaften, auch eine deutliche Verbesserung des sensorischen Gefühls der resultierenden Zubereitungen.

Als anorganische Verdickungsmittel haben sich Schichtsilikate als besonders geeignet im Sinne der vorliegenden Erfindung erwiesen. Insbesondere Tone, wie beispielsweise Bentonit, und synthetische Schichtsilikate, wie beispielsweise das von der Firma Süd Chemie unter der Handelsbezeichnung Optigel^{®} vertriebene Magnesiumschichtsilikat, sind bevorzugt.

Weiterhin hat es sich als vorteilhaft erweisen, wenn die Oxidationsmittelzubereitungen mindestens einen Stabilisator enthalten. Besonders bevorzugte Stabilisatoren sind EDTA, 1-Hydroxyethan-1,1-diphoshonsäure, Dipicolinsäure, Phenacetin, Alkalibenzoate (Natriumbenzoat), Alkalistannate (Natriumstannat), Alkalipyrophosphate (Tetranatriumpyrophosphat, Dinatriumpyrophosphat), Salicylsäure, Alkaliphosphate (Natriumphosphat), und Phosphorsäure.

Im Rahmen eines dritten Gegenstandes der vorliegenden Erfindung werden die Fasern im Rahmen des ersten Verfahrensschrittes mit einer Zubereitung behandelt, die unmittelbar vor der Anwendung aus einer ersten Zubereitung (A), enthaltend mindestens ein Oxidationsfarbstoffvorprodukt, einer zweiten Zubereitung (B), enthaltend mindestens ein Oxidationsmittel, und einer dritten Zubereitung (C), enthaltend mindestens einen Pflegestoff (P2), hergestellt wird.

Der Einsatz der erfindungsgemäßen Zubereitung (C) ermöglicht, dass das Haar bereits während der Coloration von innen aufgebaut wird und dass dem oxidativen Stress entgegengewirkt wird. Dies lässt sich an einem deutlich verbesserten Pflegezustand der Fasern nach der Behandlung erkennen.

Hinsichtlich der Bestandteile der Zubereitungen (A) und (B) sei an dieser Stelle auf die Ausführungen verwiesen, die im Rahmen der ersten bevorzugten Ausführungform gemacht wurden.

Hinsichtlich des in der Zubereitung (C) erfindungsgemäß vorzugsweise einsetzbaren mindestens einem Pflegestoff (P2) sei an dieser Stelle explizit auf die Ausführungen zum Pflegestoff (P1) verwiesen.

Obwohl prinzipiell alle Konfektionierungsformen denkbar sind, hat es sich als bevorzugt erwiesen, wenn die Zubereitung (C) in Form einer wässrigen oder wässrig-alkoholischen Lösung oder Gels formuliert wird. Bezüglich des Begriffs "wässrig" sei auf die obige Definition verwiesen. Vorzugsweise enthält die Zubereitung (C) neben dem mindestens einen Pflegestoff (P2) mindestens ein Verdickungsmittel. Auch hinsichtlich der Verdickungsmittel sei auf die obigen Ausführungen verwiesen.

Weiterhin hat es sich als bevorzugt herausgestellt, wenn die Zubereitung (C) mindestens ein Konservierungsmittel enthält. Erfindungsgemäß besonders bevorzugte Konservierungsmittel sind Phenoxyethanol, Methylparaben, Butylparaben, Ethylparaben, Propylparaben und Isobutylparaben, Benzoesäure und ihre Salze sowie Ester, Formaldehyd, Paraformaldehyd, 2-Hydroxybiphenyl, anorganische Sulfite und Bisulfite, 4-Hydroxybenzoesäure und ihre Salze und Ester, Chlorbutanol, Dehydracetatsäure, Bronidox, Bronopol, Triclosan, Benzylalkohol, Chlorkresol, Chlorxylenol und Chlorhexidin.

Im Rahmen eines vierten Gegenstandes der vorliegenden Erfindung hat es sich als bevorzugte erwiesen, wenn die Fasern unmittelbar vor der oxidativen Färbung mit einer Zubereitung (E), enthaltend mindestens einen Pflegestoff (P3), vorbehandelt werden.

Durch die Maßnahme der erfindungsgemäßen Faservorbehandlung ist es möglich, ein einheitliches Färbeergebnis vom Ansatz bis zur Spitze der Fasern unabhängig vom Grad der Vorschädigung zu erhalten.

Die Zubereitung (E) wird am Anfang des erfindungsgemäßen Färbeverfahrens auf die Fasern aufgetragen. Obwohl prinzipiell einer Anwendung auf bereits angefeuchtetem Haar nichts im Wege steht, wird die Zubereitung (E) vorzugsweise auf die trockenen Fasern aufgetragen.

Obwohl es erfindungsgemäß möglich ist, die Fasern nach Anwendung der Zubereitung (E) gründlich mit Wasser zu spülen, hat es sich als erfindungsgemäß bevorzugt erwiesen, die Zubereitung (E) nicht abzuspülen, sondern vielmehr unmittelbar nach der Einwirkzeit der Zubereitung (E) das Färbemittel aufzutragen.

Einwirkzeiten von wenigen Sekunden bis zu 30 Minuten haben sich für die Zubereitung (E) erfindungsgemäß als bevorzugt erwiesen.

Hinsichtlich der im Rahmen der Zubereitung (E) eingesetzten Pflegestoffe (P3) sei an dieser Stelle explizit auf die Ausführungen zum Pflegestoff (P1) verwiesen.
Obwohl prinzipiell keinerlei Einschränkungen hinsichtlich der Konfektionierungsform der Zubereitung (E) bestehen, hat es sich als erfindungsgemäß bevorzugt erwiesen, wenn die Zubereitung (E) als wässriges oder wässrig-alkoholisches Spray formuliert ist. Auch hier gilt die oben gegebene Definition des Begriffes "wässrig-alkoholisch".

Erfindungsgemäß können auch Zubereitungen (E) eingesetzt werden, die neben dem mindestens einen Pflegestoff (P3) mindestens einen direktziehenden Farbstoff enthalten. Hinsichtlich der erfindungsgemäß bevorzugten direktziehenden Farbstoffe sei an dieser Stelle auf die entsprechenden Ausführungen zur Zubereitung (A) verwiesen.

Im Rahmen der vorliegenden Erfindung hat es sich als besonders bevorzugt erwiesen, wenn die in den verschiedenen Stufen des erfindungsgemäßen Verfahrens eingesetzten Pflegestoffe (P1), (P2) und gegebenenfalls (P3) identisch sind.

Neben den erfindungswesentlichen Komponenten können die im Rahmen des erfindungsgemäßen Verfahren eingesetzten Zubereitungen (A), (B), (C), (D) beziehungsweise (E) unabhängig voneinander mindestens ein pflegendes Polymer enthalten.

Eine erste Gruppe der pflegenden Polymere sind die kationischen Polymere. Unter kationischen Polymeren sind erfindungsgemäß Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (G1-I), 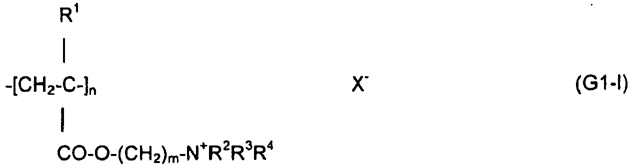 in der R¹= -H oder -CH₃ ist, R², R³ und R⁴ unabhängig voneinander ausgewählt sind aus C₁₋₄-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel (G1-I) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
Ru steht für eine Methylgruppe
R², R³ und R⁴ stehen für Methylgruppen
m hat den Wert 2.

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel (G1-I) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Weitere bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- kationische Alkylpolyglycoside gemäß der DE-PS 44 13 686,
- kationiserter Honig, beispielsweise das Handelsprodukt Honeyquat^{®} 50,
- kationische Guar-Derivate, wie insbesondere die unter den Handelsnamen Cosmedia^{®} Guar und Jaguar^{®} vertriebenen Produkte,
- Polysiloxane mit quaternären Gruppen, wie beispielsweise die im Handel erhältlichen Produkte Q2-7224 (Hersteller: Dow Corning; ein stabilisiertes Trimethylsilylamodimethicon), Dow Corning^{®} 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt), diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden,
- quaternierter Polyvinylalkohol,
- sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Weitere erfindungsgemäße kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen^{®} CMF, Hydagen^{®} HCMF, Kytamer^{®} PC und Chitolam^{®} NB/101 im Handel frei verfügbar sind.

Erfindungsgemäß bevorzugte kationische Polymere sind kationische Cellulose-Derivate und Chitosan und dessen Derivate, insbesondere die Handelsprodukte Polymer^{®}JR 400, Hydagen^{®} HCMF und Kytamer^{®} PC, kationische Guar-Derivate, kationische Honig-Derivate, insbesondere das Handelsprodukt Honeyquat^{®} 50, kationische Alkylpolyglycodside gemäß der DE-PS 44 13 686 und Polymere vom Typ Polyquaternium-37.

Weiterhin sind kationiserte Proteinhydrolysate zu den kationischen Polymeren zu zählen, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen kann. Die den erfindungsgemäßen kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder einer Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quarternären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die erfindungsgemäßen kationischen Proteinhydrolysate und -derivate seien die unter den INCI - Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte genannt: Cocodimonium Hydroxypropyl Hydrolyzed Collagen, Cocodimopnium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Soy Protein, Cocodimonium Hydroxypropyl Hydrolyzed Wheat Protein, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed Keratin, Hydroxypropyltrimonium Hydrolyzed Rice Bran Protein, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Soy Protein, Quaternium-79 Hydrolyzed Wheat Protein.

Ganz besonders bevorzugt sind die kationischen Proteinhydrolysate und -derivate auf pflanzlicher Basis.

Weitere erfindungsgemäß einsetzbare pflegende Polymere sind die amphoteren Polymere. Bevorzugte amphotere Polymer werden in der britischen Offenlegungsschrift 2 104 091, der europäischen Offenlegungsschrift 47 714, der europäischen Offenlegungsschrift 217 274, der europäischen Offenlegungsschrift 283 817 und der deutschen Offenlegungsschrift 28 17 369 genannt.

Bevorzugt eingesetzte amphotere Polymere sind solche Polymerisate, die sich im wesentlichen zusammensetzen aus
(a) Monomeren mit quartären Ammoniumgruppen der allgemeinen Formel (II),

   R¹-CH=CR²-CO-Z-(CₙH₂ₙ)-N⁽⁺⁾R³R⁴R⁵ A⁽⁻⁾ (II)

   in der R¹ und R² unabhängig voneinander stehen für Wasserstoff oder eine Methylgruppe und R³, R⁴ und R⁵ unabhängig voneinander für Alkylgruppen mit 1 bis 4 Kohlenstoffatomen, Z eine NH-Gruppe oder ein Sauerstoffatom, n eine ganze Zahl von 2 bis 5 und A⁽⁻⁾ das Anion einer organischen oder anorganischen Säure ist, und
(b) monomeren Carbonsäuren der allgemeinen Formel (III),

   R⁶-CH=CR⁷-COOH (III)

   in denen R⁶ und R⁷ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Bezüglich der Einzelheiten der Herstellung dieser Polymerisate wird ausdrücklich auf den Inhalt der deutschen Offenlegungsschrift 39 29 973 Bezug genommen. Ganz besonders bevorzugt sind solche Polymerisate, bei denen Monomere des Typs (a) eingesetzt werden, bei denen R³, R⁴ und R⁵ Methylgruppen sind, Z eine NH-Gruppe und A⁽⁻⁾ ein Halogenid-, Methoxysulfat- oder Ethoxysulfat-Ion ist; Acrylamidopropyl-trimethyl-ammoniumchlorid ist ein besonders bevorzugtes Monomeres (a). Als Monomeres (b) für die genannten Polymerisate wird bevorzugt Acrylsäure verwendet.

Die erfindungsgemäßen Zubereitungen enthalten die kationischen und/oder die amphoteren Polymere bevorzugter Weise in einer Menge von 0,01 bis 5 Gew.-%, insbesondere in einer Menge von 0,1 bis 2 Gew.-%.

Weiterhin können die im Rahmen des erfindungsgemäßen Verfahren eingesetzten Zubereitungen (A), (B), (C), (D) beziehungsweise (E) neben den erfindungswesentlichen Komponenten unabhängig voneinander mindestens ein kationisches Tensid enthalten.

Erfindungsgemäß bevorzugt sind kationische Tenside vom Typ der quarternären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75, Dehyquart^{®} C-4046, Dehyquart^{®} L80 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Eine weitere Gruppe der kationischen Tenside stellen die unter den INCI-Bezeichnungen Linoleamidopropyl PG-Dimonium Chloride Phosphate, Cocamidopropyl PG-Dimonium Chloride Phosphate und Stearamidopropyl PG-Dimonium Chloride Phosphate bekannten Substanzen dar. Diese werden beispielsweise von der Firma Mona unter den Handelsbezeichnungen Phospholipid EFA^{®}, Phospholipid PTC^{®} sowie Phospholipid SV^{®} vertrieben.

Die kationischen Tenside sind in den erfindungsgemäßen Mitteln bevorzugt in Mengen von 0,05 bis 10 Gew.-%, bezogen auf die gesamte Anwendungszubereitung, enthalten. Mengen von 0,1 bis 5 Gew.-% sind besonders bevorzugt.

Die erfindungsgemäßen Zubereitungen können weiterhin alle für solche Zubereitungen bekannten Wirk-, Zusatz- und Hilfsstoffe enthalten. In vielen Fällen hat es sich als bevorzugt erwiesen, wenn die Zubereitungen mindestens ein Tensid enthalten. In vielen Fällen hat es sich als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel R¹O-(Z)ₓ. Diese Verbindungen sind durch die folgenden Parameter gekennzeichnet.

Der Alkylrest R¹ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R¹
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, dass eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyldimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine - COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Ein Beispiel für ein als kationisches Tensid einsetzbares quaternäres Zuckerderivat stellt das Handelsprodukt Glucquat^{®}100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder - alkoholate als Katalysatoren verwendet werden. Die Verwendung von Produkten mit eingeengter Homologenverteilung kann bevorzugt sein.
Ferner können die erfindungsgemäßen Mittel weitere Wirk-, Hilfs- und Zusatzstoffe, wie beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, N₂O, Dimethylether, CO₂ und Luft,
- Antioxidantien,
enthalten

Bezüglich weiterer fakultativer Komponenten sowie die eingesetzten Mengen dieser Komponenten wird ausdrücklich auf die dem Fachmann bekannten einschlägigen Handbücher, z. B Kh. Schrader, Grundlagen und Rezepturen der Kosmetika, 2. Auflage, Hüthig Buch Verlag, Heidelberg, 1989, verwiesen.

Ein fünfter Gegenstand der vorliegenden Erfindung ist ein Kit zur schonenden Färbung keratinischer Fasern, umfassend
- eine Zubereitung (A), enthaltend mindestens ein Oxidations-vorprodukt,
- eine Zubereitung (B), enthaltend mindestens ein Oxidationsmittel, und
- eine sauer eingestellte schaumförmige Zubereitung (D), enthaltend mindestens einen Pflegestoff (P1), wobei die schaumförmige Zubereitung als Aerosol formuliert ist.

Hinsichtlich der verschiedenen Zubereitungen sei an dieser Stelle explizit auf die Ausführungen verwiesen, die bei der Beschreibung des erfindungsgemäßen Verfahrens getroffen wurden.
Im Rahmen einer bevorzugten Ausführungsform enthält das Kit weiterhin eine Zubereitung (C), die mindestens einen Pflegestoff (P2) enthält.

Vorzugsweise ist die Zubereitung (C) in Form eines Gels konfektioniert ist.

Im Rahmen einer weiteren bevorzugten Ausführungsform umfasst das Kit weiterhin ein Vorbehandlungsmittel (E), enthaltend mindestens einen Pflegestoff (P3).

Das erfindungsgemäße Vorbehandlungsmittel (E) ist vorzugsweise als Spray formuliert.

Ein sechster Gegenstand ist ein Kit zur schonenden Färbung keratinischer Fasern, umfassend
- eine Zubereitung (C), enthaltend mindestens einen Pflegestoff (P2),
- eine sauer eingestellte schaumförmige Zubereitung (D), enthaltend mindestens einen Pflegestoff (P1) und
- ein Vorbehandlungsmittel (E), enthaltend mindestens einen Pflegestoff (P3).

Zur Erläuterung dieses Gegenstandes der vorliegenden Erfindung sei auf die bereits gemachten Ausführungen zu den übrigen Gegenständen der Erfindung verwiesen.

Die folgenden Beispiele sollen den Anmeldungsgegenstand näher erläutern ohne diesen zu beschränken.

### Ausführungsbeispiele

Die im Rahmen der Beispiele angegebenen Mengenangaben verstehen sich sofern nichts anderes vermerkt ist in Gewichtsprozent.

### 1 Formulierungen

### 1.1 Vorbehandlungsmittel (E)

| Rohstoff | Menge |
|---|---|
| Dow Corning^{®} 939 | 1,0 |
| Methylparaben | 0,1 |
| Milchsäure 80% | 0,2 |
| Dehyquart^{®} L-80 | 2,0 |
| Synthalen^{®} CR | 0,3 |
| Gluadin^{®} WLM | 0,5 |
| Phenoxyethylalkohol | 0,3 |
| Panthenol 75L | 0,2 |
| Cremophor^{®} CO 40 | 0,4 |
| Ethylalkohol vergällt 96% | 15,0 |
| Parfum | 0,3 |
| Wasser | ad 100 |

### 1.2 Zusatz zur Färbecreme (C)

| Rohstoff | Menge |
|---|---|
| Tylose^{®} H 10000 | 1,0 |
| Euxyl^{®} K300 | 1,0 |
| Serin | 12,0 |
| Carnitin Tatrat | 6,0 |
| Wasser | ad 100 |

### 1.3 Schaumförmige Zubereitung (D)

| | Variante D1 | Variante D2 | Variante D3 | Variante D4 | Variante D5 | Variante D6 (Lotion) | Variante D7 |
|---|---|---|---|---|---|---|---|
| Dow Corning^{®} 939 | 0,5 | 0,5 | 0,5 | 0,5 | 0,5 | 0,5 | 0,5 |
| D-Panthenol^{®} | 0,2 | 0,2 | -- | 0,2 | 0,2 | 0,2 | 0,2 |
| Genamin^{®} CTAC | 1,2 | 1,2 | 1,2 | 1,2 | 1,2 | 1,2 | 1,2 |
| Glucolymone Fruit Concentrate^{®} | 0,2 | 0,2 | -- | 0,2 | 0,2 | 0,2 | 0,2 |
| Methylparaben | 0,2 | 0,2 | 0,2 | 0,2 | 0,2 | 0,2 | 0,2 |
| Escalol^{®} HP-610 | 0,9 | 0,9 | 0,9 | 0,9 | 0,9 | 0,9 | 0,9 |
| Vitamin E Acetat | 0,2 | 0,2 | -- | 0,2 | 0,2 | 0,2 | 0,2 |
| Salcare^{®} SC 96 | 0,9 | 0,9 | 0,9 | 0,9 | 0,9 | 0,9 | 0,9 |
| Phenoxyethanol | 0,4 | 0,4 | 0,4 | 0,4 | 0,4 | 0,4 | 0,4 |
| Isobutan | 8,0 | 8,0 | 8,0 | 8,0 | 8,0 | -- | 8,0 |
| Carnitin Tatrat | -- | 0,1 | 0,1 | -- | -- | 0,1 | 0,1 |
| Crodarom Pearl^{®} | -- | -- | -- | 0,5 | -- | 0,5 | 0,5 |
| Serin | -- | -- | -- | -- | 0,5 | -- | -- |
| Parfum | 0,2 | 0,2 | 0,2 | 0,2 | 0,2 | 0,2 | 0,2 |
| pH | 3,8 | 3,8 | 3,8 | 3,8 | 3,8 | 3,8 | 3,8 |
| Wasser | ad 100 | ad 100 | ad 100 | ad 100 | ad 100 | ad 100 | ad 100 |

### 1.4 Wasserstoffperoxidzubereitung

| Rohstoff | Menge |
|---|---|
| Dipicolinsäure | 0,1 |
| 50%ige KOH | 0,3 |
| Natriumbenzoat | 0,04 |
| Natriumpyrophosphat | 0,1 |
| Turpinal^{®} SL | 0,4 |
| 1,2-Propylenglykol | 0,4 |
| Cetyl/Stearylalkohol 50 : 50 | 4,0 |
| Dehyquart^{®} B | 0,75 |
| Eumulgin^{®} B2 | 1,2 |
| Paraffinöl | 0,3 |
| 50%ige H₂O₂ | 12,2 |
| Wasser | ad 100,0 |

### 1.5 Färbecreme (A)

| | Variante A1 | Variante A2 | Variante A3 |
|---|---|---|---|
| Ammoniumcarbopollsg. | 15,0 | -- | 15,0 |
| Carbomer 0,2% | -- | 0,15 | -- |
| Ammoniumrohagitlsg. | 5,0 | -- | -- |
| Eumulgin^{®} KE 2602 | 6,0 | -- | -- |
| Eumulgin^{®} B2 | -- | 3,0 | 3,0 |
| Eutanol^{®} G | -- | 2,0 | 2,0 |
| Kaliumoleinseife 12,5% | 15,0 | 3,0 | -- |
| Kalium-Ricinus-Seife 12,5% | 3,0 | -- | -- |
| Natriumlauylethersulfat 27% | -- | 4,4 | 4,4 |
| Plantacare^{®} 2000 UP | 0,5 | -- | -- |
| Titandioxid | 0,5 | 0,5 | 0,5 |
| Fettalkoholsulfat-Na | | 0,5 | -- |
| Cetiol^{®} V | 3,0 | -- | -- |
| Lorol^{®} C16 | 16,0 | -- | -- |
| Cutina^{®} GMS-V | 3,0 | -- | -- |
| Cutina^{®} GMS SE | -- | 1,0 | 2,0 |
| Cutina^{®} AGS | -- | 2,0 | 2,0 |
| Cetearyl Alcohol | -- | 10,2 | 12,0 |
| Polyglycol 600 | -- | 0,6 | -- |
| Phospholipid^{®} EFA | 1,0 | 0,1 | 0,1 |
| Kaliumhydroxyid 50% | 0,5 | 0,95 | 0,7 |
| Tetranatrium EDTA | 0,4 | 0,2 | 0,2 |
| Ammoniak 25% | 6,0 | -- | 8,0 |
| Monoethanolamin | -- | 3,0 | -- |
| Ascorbinsäure | 0,05 | 0,05 | 0,1 |
| Natriumsulfit | 0,2 | 0,2 | 0,25 |
| Merquat Plus^{®} 3330 | -- | 1,5 | 1,5 |
| Silsoft^{®} A-843 | -- | 0,5 | -- |
| Mirapol^{®} A-15 | 0,2 | -- | -- |
| Puricare^{®} LS 9658 | -- | -- | 1,0 |
| Parfum | 0,5 | 0,15 | 0,4 |
| Aerosile^{®} | 0,25 | -- | -- |
| p-Toluendiaminsulfat | 0,3 | 1,2 | 1,5 |
| 1-(2-Hydroxyethyl)-4,5-diaminopyrazol-sulfat | 0,5 | -- | -- |
| 4-Amino-3-Metylphenol | 0,3 | -- | -- |
| 2-Metylresorcin | 0,04 | 0,18 | -- |
| 1-Naphtol | 0,05 | -- | 0,02 |
| Resorcin | 0,03 | 0,3 | -- |
| m-Aminophenol | -- | 0,1 | 0,3 |
| 2-Amino-3-hydroxypyridin | 0,13 | 0,05 | 0,2 |
| 5-Amino-2-methylphenol | 0,14 | 0,01 | 0,01 |
| 4-Clorresorcin | -- | -- | 0,1 |
| 2-Amino-6-chloro-4-nitrophenol | 0,3 | 0,1 | -- |
| 4-Amino-3-nitophenol | -- | -- | 0,1 |
| wasser | ad 100 | ad 100 | ad 100 |

### 2 Vergleichsversuche

Auf das trockene Kopfhaar von 15 Probandinnen wurde das Vorbehandlungsmittel E (Rezeptur siehe Punkt 1.1) aufgetragen. Nach einer Einwirkzeit von 2 Minuten wurde auf die Haare eine Färbezubereitung aufgetragen, die unmittelbar vor der Anwendung aus einer Mischung von 30 g Färbecreme A3, 30 g Oxidationsmittelzubereitung (Rezeptur siehe Punkt 1.4) und 0,5 g Booster (Rezeptur siehe Punkt 1.2) erhalten wurde. Nach einer Einwirkzeit wurden die Haare in verschiedene Bereiche aufgeteilt und behandelt, wie in der folgenden Abbildung gezeigt (Aufsicht auf den Kopf einer Probandin):

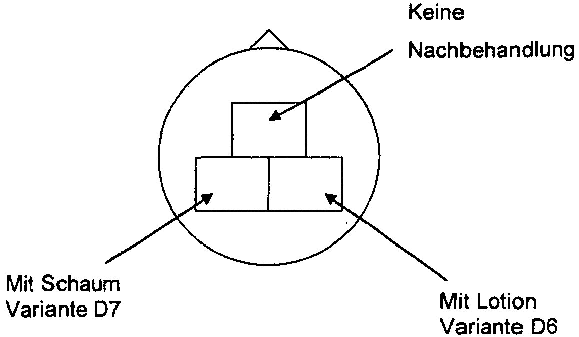

Nach weiteren 5 Minuten wurden die Haare gründlich gespült.

Anschließend wurde in allen Fällen das Färbeergebnis von 5 besonders geschulten Personen beurteilt, das heißt die Färbleistung in den drei gekennzeichneten Haarbereichen verglichen.
Die Haare von jeweils insgesamt 6 der Probandinnen wurden nach 6 normalen Waschzyklen im alltäglichen Leben erneut beurteilt.
Das Ergebnis der Untersuchungen ist in der folgenden Tabelle wiedergegeben, dabei verstehen sich die Werte jeweils als Beurteilung in Bezug zu den ohne Nachbehandlung gefärbten Haaren (vorderer Bereich):

| | Schaum | | Lotion (Spray) | |
|---|---|---|---|---|
| Farbergebnis | +: | 10 | +: | 3 |
| | 0: | 4 | 0: | 7 |
| | -: | 1 | -: | 5 |
| Abtragung nach ca. 6 Haarwäschen | +: | 4 | +: | 1 |
| | 0: | 1 | 0: | 3 |
| | -: | 1 | -: | 2 |
| Pflegeeigenschaften | +: | 10 | +: | 7 |
| | 0: | 3 | 0: | 6 |
| | -: | 2 | -: | 2 |
| + besseres Ergebnis mit Nachbehandlung durch Schaum beziehungsweise Lotion | | | | |
| 0 gleiches Ergebnis mit und ohne Nachbehandlung durch Schaum beziehungsweise Lotion | | | | |
| - schlechteres Ergebnis mit Nachbehandlung durch Schaum beziehungsweise Lotion | | | | |

Anhand der Resultate lässt sich deutlich ablesen, dass die Pflegezustand der mit dem Schaum behandelten Haare deutlich besser beurteilt wurde, als der mit der Lotion behandelten Haare Auch das Farbergebnis unmittelbar nach der Farbbehandlung und nach 6 Haarwäschen wird für die mit dem Schaum behandelten Haaren deutlich besser beurteilt.

### 3 Verzeichnis der eingesetzten Handelsprodukte

| | |
|---|---|
| Cremophor^{®} CO40 | hydriertes Rizinusöl mit ca. 40-45 EO-Einheiten (INCI-Bezeichnung: PEG-40 Hydrogenated Castor Oil) (BASF) |
| Crodarom^{®} Pearl | Perlenextrakt (ca. 48-54% Wassergehalt; INCI-Bezeichnung: Aqua (Water), Glycerin, Pearl Powder, Maris Sal (Sea Salt)) (Crodarom) |
| Dehyquart^{®} B | Stearyltrimethylammoniumchlorid (ca. 60-66% Aktivsubstanzgehalt; INCI-Bezeichnung: Steartrimonium Chloride) (Cognis) |
| Dehyquart^{®} L80 | Bis(Cocoylethyl)-hydroxyethyl-methyl-ammonium-methosulfat (ca. 76 % Aktivsubstanz in Propylenglykol; INCI-Bezeichnung: Dicocoylethyl Hydroxyethylmonium Methosulfat, Propylene Glycol) (Henkel) |
| Dow Corning^{®} 939 | ca. 32-36% Festkörper; INCI-Bezeichnung: Amodimethicone, Trideceth-12, Cetrimonium Chloride (Dow Corning) |
| Escalol^{®} HP-610 | ca. 12-18% Wassergehalt, min. 65 % QAV-Anteil; INCI-Bezeichnung: Dimethylpabamidopropyl Laurdimonium Tosylate, Propylene Glycol Stearate (ISP Global) |
| Eumulgin^{®} B2 | Cetylstearylalkohol mit ca. 20 EO-Einheiten (INCI-Bezeichnung: Ceteareth-20) (Cognis) |
| Euxyl^{®} K 300 | Mischung aus Phenoxyethanol, Methylparaben, Butylparaben, Ethylparaben, Propylparaben und Isobutylparaben (Schülke & Mayr) |
| Genamin^{®} CTAC | Trimethylhexadecylammoniumchlorid (ca. 28-30% Aktivsubstanz in Wasser; INCI-Bezeichnung: Cetrimonium Chloride) (Clariant) |
| Gluadin^{®} WLM | Weizenproteinhydrolysat (ca. 21-24% Festkörper; INCI-Bezeichnung: Hydrolyzed Wheat Protein) (Cognis) |
| Glucolymone Fruit Concentrate^{®} | ca. 20-30% Trockensubstanzgehalt (INCI-Bezeichnung: Aqua (Water), Propylene Glycol, Lemon (Citrus Medica Limonum) Exract, Dextrin, Grapefruit (Citrus Grandis) Extract) (Vincience) |
| Salcare^{®} SC 96 | ca. 50% Aktivsubstanzgehalt; INCI-Bezeichnung: Polyquaternium-37, Propylene Glycol Dicaprylate/Dicaprate, PPG-1 Trideceth-6 (CIBA) |
| Synthalen^{®} CR | 2-(Trimethylammonio)ethylmethacrylatchloridhomopolymer (INCI-Bezeichnung: Polyquaternium-37) (3V Sigma) |
| Turpinal^{®} SL | 1-Hydroxyethan-1,1-diphosphonsäure (ca. 58 - 61% Aktivsubstanzgehalt; INCI-Bezeichnung: Etidronic Acid, Aqua (Water)) (Solutia) |
| Tylose^{®} H 10000 | Hydroxyethylcellulose (Clariant) |

## Patentansprüche

1. Verfahren zur schonenden Färbung keratinischer Fasern, insbesondere menschlicher Haare, bei dem
- in einem ersten Schritt die Fasern einer oxidativen Färbung unterzogen werden und
- anschließend in einem zweiten Schritt mit einer sauer eingestellten schaumförmigen Zubereitung, enthaltend mindestens einen Pflegestoff (P1), behandelt werden,
**dadurch gekennzeichnet, dass** die schaumförmige Zubereitung als Aerosol formuliert ist.

2. Verfahren nach Anspruch 1, **dadurch gekennzeichnet, dass** im Rahmen des ersten Verfahrensschrittes die Fasern mit einer Zubereitung behandelt werden, die unmittelbar vor der Anwendung aus einer ersten Zubereitung (A), enthaltend mindestens ein Oxidationsfarbstoffvorprodukt, und einer zweiten Zubereitung (B), enthaltend mindestens ein Oxidationsmittel, hergestellt wird.

3. Verfahren nach Anspruch 1, **dadurch gekennzeichnet, dass** im Rahmen des ersten Verfahrensschrittes die Fasern mit einer Zubereitung behandelt werden, die unmittelbar vor der Anwendung aus einer ersten Zubereitung (A), enthaltend mindestens ein Oxidationsfarbstoffvorprodukt, einer zweiten Zubereitung (B), enthaltend mindestens ein Oxidationsmittel, und einer dritten Zubereitung (C), enthaltend mindestens einen Pflegestoff (P2), hergestellt wird.

4. Verfahren nach einem der Ansprüche 1 bis 3, **dadurch gekennzeichnet, dass** die Fasern unmittelbar vor der oxidativen Färbung mit einer Zubereitung (E), enthaltend mindestens einen Pflegestoff (P3), vorbehandelt werden.

5. Verfahren nach Anspruch 4, **dadurch gekennzeichnet, dass** die Zubereitung (E) mindestens einen direktziehenden Farbstoff enthält.

6. Verfahren nach einem der Ansprüche 1 bis 5, **dadurch gekennzeichnet, dass** die Pflegestoffe (P1), (P2) und/oder (P3) unabhängig voneinander ausgewählt sind aus Carnitin und seinen Salzen, insbesondere Carnitintatrat, Aminosäuren, insbesondere Serin, Lysin und Glycin, Extrakten, die aus Perlen gewonnen werden können, Bestandteilen, die aus Edelsteinen gewonnen werden können, Meersalzmineralien, Pflanzenölen, Pflanzenextrakten und/oder Vitaminen.

7. Verfahren nach einem der Ansprüche 1 bis 6, **dadurch gekennzeichnet, dass** die in den drei Stufen eingesetzten Pflegestoffe (P1), (P2) und (P3) identisch sind.

8. Kit zur schonenden Färbung keratinischer Fasern, umfassend
- eine Zubereitung (A), enthaltend mindestens ein Oxidationsfarbstoff-vorprodukt,
- eine Zubereitung (B), enthaltend mindestens ein Oxidationsmittel, und
- eine sauer eingestellte schaumförmige Zubereitung (D), enthaltend mindestens einen Pflegestoff (P1), **dadurch gekennzeichnet, dass** die schaumförmige Zubereitung als Aerosol formuliert ist.

9. Kit nach Anspruch 8, **dadurch gekennzeichnet, dass** es weiterhin eine Zubereitung (C) enthält, die mindestens einen Pflegestoff (P2) enthält.

10. Kit nach Anspruch 9, **dadurch gekennzeichnet, dass** die Zubereitung (C) in Form eines Gels konfektioniert ist.

11. Kit nach einem der Ansprüche 8 bis 10, **dadurch gekennzeichnet, dass** es weiterhin ein Vorbehandlungsmittel (E), enthaltend mindestens einen Pflegestoff (P3), umfasst.

12. Kit nach Anspruch 11, **dadurch gekennzeichnet, dass** das Vorbehandlungsmittel als Spray formuliert ist.

## Claims

1. A method for gently coloring keratin fibers, in particular human hair, wherein,
- in a first step, the fibers are subjected to oxidative coloring and
- then, in a second step, treated with a foamed preparation which has been made acidic and contains at least one nourishing substance (P1),
**characterized in that** the foamed preparation is formulated as an aerosol.

2. The method according to claim 1, **characterized in that**, in the context of the first method step, the fibers are treated with a preparation which is made immediately before use from a first preparation (A), containing at least one oxidation dye precursor, and a second preparation (B), containing at least one oxidizing agent.

3. The method according to claim 1, **characterized in that**, in the context of the first method step, the fibers are treated with a preparation which is made immediately before use from a first preparation (A), containing at least one oxidation dye precursor, a second preparation (B), containing at least one oxidizing agent, and a third preparation (C), containing at least one nourishing substance (P2).

4. The method according to one of claims 1 to 3, **characterized in that**, immediately before oxidative coloring, the fibers are pretreated with a preparation (E) containing at least one nourishing substance (P3).

5. The method according to claim 4, **characterized in that** the preparation (E) contains at least one direct dye.

6. The method according to one of claims 1 to 5, **characterized in that** the nourishing substances (P1), (P2) and/or (P3) are selected independently of one another from carnitine and salts thereof, in particular carnitine tartrate, amino acids, in particular serine, lysine and glycine, extracts that can be obtained from pearls, components that can be obtained from gemstones, sea salt minerals, plant oils, plant extracts and/or vitamins.

7. The method according to one of claims 1 to 6, **characterized in that** the nourishing substances (P1), (P2) and (P3) used in the three steps are identical.

8. A kit for gently coloring keratin fibers, comprising
- a preparation (A), containing at least one oxidation dye precursor,
- a preparation (B), containing at least one oxidizing agent, and
a foamed preparation (D) which has been made acidic and contains at least one nourishing substance (P1),
**characterized in that** the foamed preparation is formulated as an aerosol.

9. The kit according to claim 8, **characterized in that** it further contains a preparation (C), which contains at least one nourishing substance (P2).

10. The kit according to claim 9, **characterized in that** the preparation (C) is produced in the form of a gel.

11. The kit according to one of claims 8 to 10, **characterized in that** it further comprises a pretreatment agent (E), containing at least one nourishing substance (P3).

12. The kit according to claim 11, **characterized in that** the pretreatment agent is formulated as a spray.

## Revendications

1. Procédé de coloration délicate de fibres de kératine, en particulier de cheveux humains, dans lequel :
- dans une première étape, les fibres sont soumises à une coloration oxydative, et
- ensuite, dans une deuxième étape, elles sont traitées par une préparation moussante rendue acide, contenant au moins un produit de soin (P1),
**caractérisé en ce que** la préparation moussante est formulée en aérosol.

2. Procédé selon la revendication 1, **caractérisé en ce que**, dans le cadre d'une première étape de procédé, les fibres sont traitées par une préparation confectionnée immédiatement avant utilisation à partir d'une première préparation (A) contenant au moins un précurseur de colorant d'oxydation et d'une deuxième préparation (B) contenant au moins un oxydant.

3. Procédé selon la revendication 1, **caractérisé en ce que**, dans le cadre d'une première étape de procédé, les fibres sont traitées par une préparation confectionnée immédiatement avant utilisation à partir d'une première préparation (A) contenant au moins un précurseur de colorant d'oxydation, d'une deuxième préparation (B) contenant au moins un oxydant et d'une troisième préparation (C) contenant au moins un produit de soin (P2).

4. Procédé selon une des revendications 1 à 3, **caractérisé en ce que** les fibres sont prétraitées immédiatement avant la coloration oxydative avec une préparation (E) contenant au moins un produit de soin (P3).

5. Procédé selon la revendication 4, **caractérisé en ce que** la préparation (E) contient au moins un colorant direct.

6. Procédé selon une des revendications 1 à 5, **caractérisé en ce que** les produits de soin (P1), (P2) et/ou (P3) sont choisis, indépendamment les uns des autres, parmi la carnitine et ses sels, en particulier le tartrate de carnitine, des acides aminés, en particulier la sérine, la lysine et la glycine, des extraits qu'on peut obtenir à partir de perles, de composants qu'on peut obtenir à partir de pierres précieuses, des sels minéraux marins, des huiles végétales, des extraits végétaux et/ou des vitamines.

7. Procédé selon une des revendications 1 à 6, **caractérisé en ce que** les produits de soin (P1), (P2) et (P3) utilisés aux deux étapes sont identiques.

8. Kit de coloration délicate de fibres de kératine comprenant :
- une préparation (A) contenant au moins un précurseur de colorant d'oxydation,
- une préparation (B) contenant au moins un colorant d'oxydation, et
- une préparation moussante rendue acide (D) contenant au moins un produit de soin (P1),
**caractérisé en ce que** la préparation moussante est formulée en aérosol.

9. Kit selon la revendication 8, **caractérisé en ce qu'**il contient en outre une préparation (C) qui contient au moins un produit de soin (P2).

10. Kit selon la revendication 9, **caractérisé en ce que** la préparation (C) est confectionnée sous forme de gel.

11. Kit selon une des revendications 8 à 10, **caractérisé en ce qu'**il contient en outre une préparation de prétraitement (E) contenant au moins un produit de soin (P3).

12. Kit selon la revendication 11, **caractérisé en ce que** le produit de prétraitement est formulé en spray.
